# EUROPEAN PATENT SPECIFICATION

(11) **EP 0 490 892 B1**
(45) Date of publication and mention of the grant of the patent: **13.11.1996**
(21) Application number: 89910959.9
(22) Date of filing: 08.09.1989
(51) Int. Cl.: A61N 1/365, A61N 1/368

(54) **PMT DETECTING PACEMAKER**
SCHRITTMACHER MIT NACHWEIS EINER VOM SCHRITTMACHER AUSGELÖSTEN TACHYKARDIE
STIMULATEUR CARDIAQUE DETECTANT UNE TACHYCARDIE INDUITE PAR LE STIMULATEUR CARDIAQUE

(43) Date of publication of application: 24.06.1992
(73) Proprietor: Pacesetter AB, 171 95 Solna (SE)
(72) Inventor: ELMQVIST, Hakan, S-161 38 Bromma (SE)
(74) Representative: Lettström, Richard Wilhelm
(86) International application number: SE8900484
(87) International publication number: WO9103274

(56) References cited:
- US-A- 4 515 161
- US-A- 4 569 350
- US-A- 4 577 634

## Description

The present invention relates to a heart pacemaker, and in particular to a heart pacemaker comprising means for detecting a pacemaker-mediated tachycardia.

Atrial synchronized pacing systems include an atrial (P-wave) sensing circuit, which, in connection with retrograde (ventriculoatrial, VA) heart tissue electrical conduction might cause a so-called pacemaker-mediated tachycardia (PMT). A PMT results when the atrial sensing circuit detects a P-wave induced by e.g. a retrogradely conducted ventricular activity outside the sensing circuit's refractory periods and the pacemaker subsequently initiates a paced ventricular beat. Repeated stimulation is sustained by heart tissue retrograde conduction and by pacemaker antegrade conduction.

Although this complication is related to atrial synchronized systems in general, it can be exemplified with reference to dual chamber (DDD) pacemakers. With the advent of the first generation of such pacemakers with relatively short atrial refractory periods, PMT was identified as a significant problem, and later generations of such pacemakers therefore include methods for preventing PMT:s.

One such known method involves the use of programmable atrial refractory periods, where the atrial refractory period is programmed to be longer than the retrograde conduction interval. Such a method is described in US-A-4 569 350 which shows a heart pacemaker comprising sensing means for detecting atrial events, stimulating means for stimulating the ventricle, variable delay means for generating a variable time delay between the detection of atrial events and the stimulation of the ventricle, time-measuring means for measuring the interval between consecutive signals of which at least one is generated by the heart and decision means for deciding that a pacemaker mediated tachycardia is present.

The system comprises the steps of sensing electrical signals from the atrium and sensing electrical signals from the ventricle. A stimulating pulse is provided to the ventricle, at a first predetermined time after an electrical signal from the atrium is sensed, if no electrical signal from the ventricle is sensed within the first predetermined time. A threshold rate for the interval between sensed electrical signals from the atrium is selected. A detection is made whether the threshold rate is exceeded for a selected number of electrical signals from the atrium. If the threshold rate is exceeded for the selected number of electrical signals from the atrium, then the first predetermined time is increased by a second predetermined time. A determination is then made whether the interval between sensed electrical signals from the atrium has increased by the second predetermined time. If the interval between sensed electrical signals from the atrium has increased by the second predetermined time, this indicates that pacemaker mediated tachycardia has occurred. However, this method lowers the upper synchronized rate limit of the pacemaker and, in patients with a long retrograde conduction interval, the refractory period required is so long that the advantages with dual chamber pacing is seriously affected.

Another known method is based on the fact that the majority of PMT:s are initiated by ventricular premature beats (VPB), viz. a ventricular event not preceded by an atrial beat. Therefore, this known method foresees that only a VPB triggers a prolonged atrial refractory period. Alternatively, a VPB could trigger a simultaneous atrial stimulation causing the atrium to be refractory when the retrograde conduction occurs. However, although this method generally allows shorter refractory periods, PMT:s remain a problem in patients where the initiating mechanism for PMT:s is unknown or is different from a VPB (Spontaneous Endless Loop Tachycardia by Oseran et al, PACE vol. 9, May-June 1986, pages 379 to 386).

Further, it is known that a PMT, once established, can for instance be terminated by omitting ventricular beats or delivering critically timed ventricular premature beats. Here, however, PMT detection is critical, and a possible detection criterion such as a stable high frequency at, or in the vicinity of, the upper rate pacing limit cannot be reliably related to a PMT (Merits of Various Antipacemaker Circus Movement Tachycardia Features by den Dulk et al, PACE, vol. 9, Nov-Dec, Part II 1986).

The object of the present invention is therefore to provide for a reliable PMT detecting device.

The invention is defined in claim 1 and preferable embodiments thereof are defined in the dependent claims.

In order that the invention may be more readily understood, an example of the invention will be described below with reference to the accompanying drawings.

Fig. 1 is a functional block diagram of the pacemaker as connected to the heart.

Fig. 2 is a functional block diagram of the pacemaker basic timing and logic unit 15.

Fig. 3 is a functional block diagram of the calculating unit 16.

Fig. 4 is a timing diagram illustrating various heart and pacer events as well as various time intervals relevant to the invention.

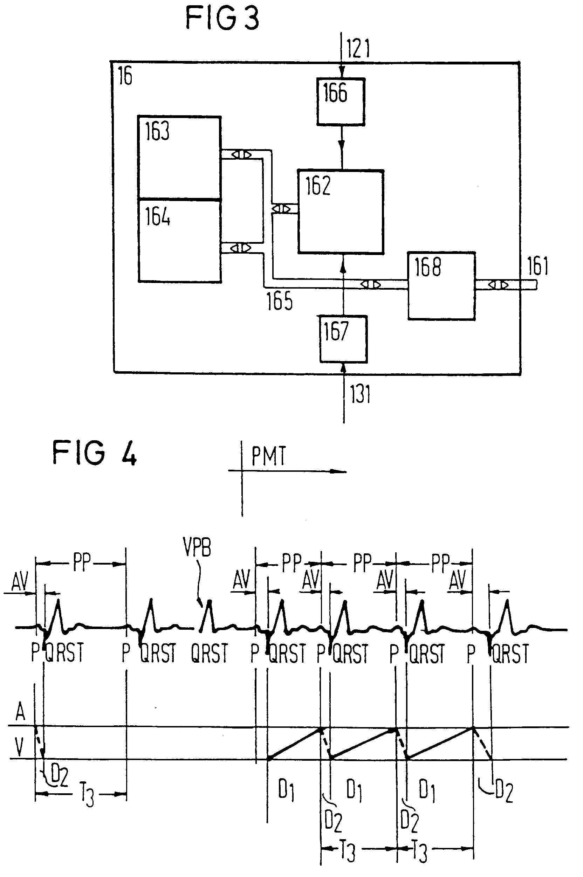

According to the invention, the pacemaker investigates the presence of a PMT by measuring the correlation between an interval T₃, for signals generated by the heart and an interval D₂, for signals generated by the pacemaker. Reference is now made to Fig. 4, where a non-PMT situation is illustrated to the left and a PMT situation is illustrated to the right. The lower part of the figure illustrates the atrial (A) and the ventricular (V) conduction levels and the dashed and the continuous lines indicate antegrade and retrograde conduction, respectively.

The cycle time of a PMT comprises two delay intervals, one (D₁) related to the retrograde heart tissue conduction, and the other one (D₂) related to the pacemaker introduced interval or time delay between atrial and ventricular activity (AV-interval). D₁ can be stable, vary regularly or stocastically. However, for short time periods, D₁ is limited and substantially constant. The AV-interval, D₂, is in this invention made to vary stocastically or in accordance with a predetermined pattern.

By varying the interval D₂ in a known way, the correlation between D₂ and T₃ is calculated for a short time period. Typically 2 to 6 heart cycles are needed to reach a reasonably safe decision that the value of the correlation exceeds a predetermined value and, consequently, that a PMT is present.

The high correlation for D₂ and T₃ when a PMT is present is explained by way of example below. Let the interval T₃ be the interval (PP) between two consecutive P-waves. If no PMT is present, the PP-interval is the interval between two spontaneous atrial beats, and the correlation between D₂ and T₃ is low as the PP-interval variation is independent of D₂. If, in contrast, a PMT is present, the PP-interval is the sum of the retrograde conduction interval D₁ and the pacemaker generated interval D₂. Consequently, during a PMT, there is a very high correlation between D₂ and T₃ (the PP-interval).

Alternatively, instead of using the PP-interval as T₃, quantities related thereto, for instance the atrial frequency or the interval between a ventricular stimulus and a P-wave, could be employed. In the last case, it is obvious that the expression "signals generated by the heart", previously used in connection with T₃, is intended to comprise signals related to ventricular stimuli, and further, that the correlation between D₂ and T₃ is contrary to where T₃ denotes the PP-interval.

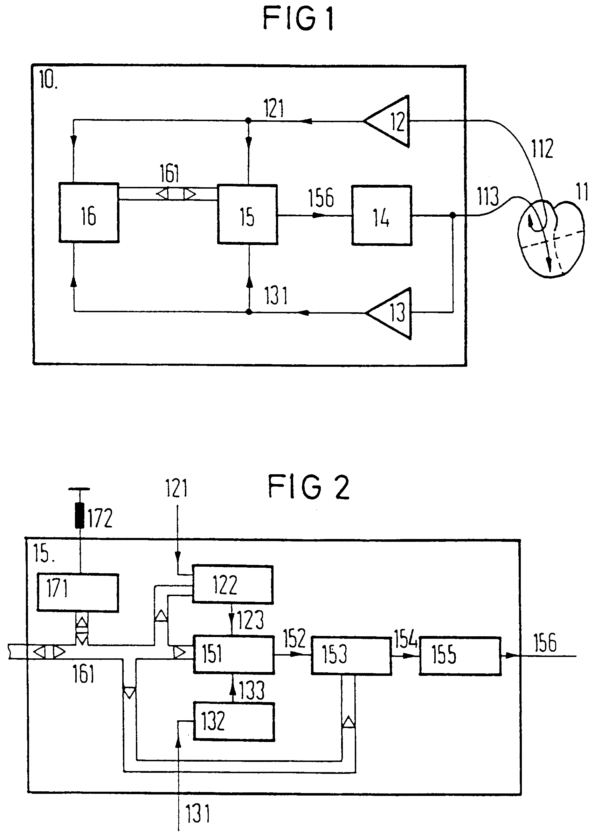

In Fig. 1, the heart is designated 11 and the pacemaker is generally designated 10. The pacemaker 10 is connected to the heart through an electrode lead 112 for the atrium and an electrode lead 113 for the ventricle. The electrode leads 112, 113 are connected to signal sensors and amplifiers 12, 13 for the atrium and the ventricle, respectively. The electrode lead (or possibly a further, not indicated separate electrode lead) for the ventricle 113, is connected to a stimulating unit 14. The stimulating unit 14 delivers stimuli to the heart. A not indicated, further stimulating unit, could possibly be provided for stimulating the atrium through electrode lead 112, or, another also not indicated, separate electrode lead. The basic pacemaker timing and logic unit 15 is controlled by the sensed signals applied thereto through leads 121 and 131. The sensed signals are related to spontaneous heart activities, viz. atrial P-wave or ventricular R-wave. If the heart fails to beat normally, stimulation pulses are emitted by the pacemaker in order to maintain the normal heart function. It is also possible to deliver correctly timed stimulation pulses even if spontaneous heart beats exist. The stimulation unit 14 is connected to the basic unit 15 through lead 156. The basic logic and timing unit 15 (to be described later) is connected to the calculating unit 16 (to be described later) through data bus 161.

In response to e.g. a sensed atrial signal on lead 121, the basic timing and logic unit 15 (Fig. 2) generates basic pacer escape intervals (PP-intervals) in the time base generator and time base register 151. A control signal on lead 152 triggers the AV-interval counter 153 simultaneously with the triggering of the time base generator 151. After the AV-interval has been timed out, a control signal on lead 154 triggers the stimulation pulse width generator and time register 155, generating a control signal on lead 156, which controls the stimulating unit 14. A further control circuit, similar to the one just described and also starting from the time base generator 151 but delivering stimuli for the atrium through electrode lead 112 or another separate (not indicated) electrode lead for the atrium can also be provided.

In order to prevent incorrect control due to false signal sensing after for instance a stimulation pulse has been delivered or a heart signal just sensed, the refractory period registers 122, 132 and their leads 123, 133 are provided in connection with atrial and ventricular signal sensing, respectively.
A communication and data register unit 171 is provided for the pacemaker programming and functional control. The communication is preferably carried out by telemetry means 172. The data bus 161 provides for the internal pacemaker transmission of programmed parameter values, control signals and time register values.

The calculating unit 16 (Fig. 3) comprises time registers, logic circuits and arithmetic processing circuits.

Preferably, a microprocessor is employed, and the microprocessor is operated in accordance with a correlation calculating program. Arithmetic processing, time measurement and control are then carried out from a RAM and ROM program memory 163 connected to a central processing unit (CPU) 162. Measured time intervals (D₂, T₃) and calculation results are stored in read-write memory 164. Detected atrial and ventricular signals are received via interrupt registers 166, 167, respectively. Data from the microprocessor can be placed in the input-output register 168 via the internal bus 165 for subsequent communication with the basic timing and logic unit 15.

It should be noted that, for the sake of explanation, the timing and logic unit 15 and the calculating unit 16 have been disclosed as separate units. It is, however, perfectly possible to arrange unit 16 to carry out the functions of timing and logic unit 15.

The calculation of the correlation is carried out in accordance with well-known mathematical theory and can be made in different ways.

By way of example, the mean value and the deviation from the mean value is calculated for each interval T₃ and each interval D₂, respectively. The deviation for each interval T₃ and for each corresponding interval D₂ are multiplied and the products added for all T₃, D₂ intervals. The resulting sum is then divided by the sum of the absolute values of the deviation for the D₂ intervals. If the result of this division exceeds a predetermined value, a PMT is present.

The PMT decision can also be referred to two correlation levels. Below the lowest level, there is no PMT and above the highest, a PMT is present. A value between the two levels indicates that a decision cannot be made with the intervals available, and therefore further intervals D₂, T₃ should be included until a decision can be reached.

Another way of calculating the correlation would be to subtract D₂ from T₃ for each corresponding D₂, T₃ interval, calculate the mean value for the resulting differences and the deviation therefrom for each resulting difference. Correspondingly, the mean value and the deviations therefrom for the intervals D₂ are calculated. These last deviations are compared to the corresponding difference deviations, and, if substantially equal, the correlation is high.

Finally, for a reliable detection of a PMT, the variation in D₂ should be of the same magnitude or greater than the variation in D₁. However, the number of intervals are also of importance for the reliability, and should the variation in D₁ be greater than that in D₂, the number of intervals should be correspondingly increased.

## Claims

1. A heart pacemaker (10) comprising at least sensing means (12) for detecting atrial events; stimulating means (14) for stimulating the ventricle; variable delay means (153) for generating a variable time delay between the detection of atrial events and the stimulation of the ventricle; time measuring means (16) for measuring the interval between consecutive signals of which at least one is generated by the heart; decision means (16) for at least deciding that a pacemaker mediated tachycardia is present, **characterized** by calculating means (16) for calculating the correlation between said time delay and said measured time interval; and that said decision means (16) decides that a pacemaker mediated tachycardia is present whenever the value of said correlation deviates from a first predetermined value.

2. A pacemaker according to claim 1, **characterized** in that said decision means (16) decides that a pacemaker mediated tachycardia is present whenever the value of said correlation exceeds said first predetermined value.

3. A pacemaker according to claim 1 and 2, **characterized** in that said decision means (16) further decides that a pacemaker-mediated tachycardia is not present when the value of said correlation falls below a second predetermined value which is lower than said first predetermined value.

4. A pacemaker according to any of the preceding claims, **characterized** in that said variable delay means (153) are arranged to vary said delay in accordance with a predetermined/programmable pattern.

5. A pacemaker according to claim 4, **characterized** in that said correlation calculation is carried out for at least two heart cycles.

6. A pacemaker according to any of the preceding claims, **characterized** in that the measured time interval is the interval between consecutive P-waves.

7. A pacemaker according to claims 5-6, **characterized** in that said correlation calculation is extended to include time delays and time measurements for further heart cycles when the value of the correlation falls between said first and second predetermined values.

8. A pacemaker according to any of the preceding claims, **characterized** in that the calculating means (16):
- calculate the mean value for the time delays and measured time intervals and the deviation from the mean value for each time delay and each measured time interval, respectively;
- multiply the deviations for each time delay and each corresponding measured time interval and add the products for all time delays and measured time intervals to a resulting sum;
- calculate the sum of the absolute values of said deviations of said time delays;
- divide said resulting sum by said sum of absolute values.

9. A pacemaker according to claim 8, **characterized** in that the measured time interval is the interval between consecutive ventricular stimulation pulses and P-waves, and that said decision means (16) decides that a pacemaker mediated tachycardia is present whenever said correlation value falls below said first predetermined value.

10. A pacemaker according to claim 9, **characterized** in that said decision means (16) further decides that a pacemaker-mediated tachycardia is not present when the value of said correlation exceeds a second value which is higher than said first predetermined value.

## Patentansprüche

1. Ein Herzschrittmacher (10) mindestens mit Abfühlmitteln (12) zum Detektieren atrieller Ereignisse; Stimulationsmitteln (14) zur Stimulation des Ventrikels; variablen Verzögerungsmitteln (153) zum Erzeugen einer variablen Zeitverzögerung zwischen der Detektion eines atriellen Ereignisses und der Stimulation des Ventrikels; Zeitmeßmitteln (16) zum Messen des Intervalls zwischen aufeinanderfolgenden Signalen, von denen wenigstens eins durch das Herz erzeugt wird; Entscheidungsmitteln (16) um zumindest zu entscheiden, daß eine herzschrittmacherverursachte Tachykardie vorliegt, **gekennzeichnet durch** Berechnungsmittel (16) zum Berechnen der Korrelation zwischen der Zeitverzögerung und dem gemessenen Zeitintervall; und daß die Entscheidungsmittel (16), entscheiden, daß eine herzschrittmacherverursachte Tachykardie immer dann vorliegt, wenn der Wert der Korrelation von einem ersten vorgegebenen Wert abweicht.

2. Ein Herzschrittmacher nach Anspruch 1, **dadurch gekennzeichnet**, daß die Entscheidungsmittel (16) entscheiden, daß eine herzschrittmacherverursachte Tachykardie immer dann vorliegt, wenn der Wert der Korrelation den ersten vorgegebenen Wert übersteigt.

3. Ein Herzschrittmacher nach Anspruch 1 und 2, **dadurch gekennzeichnet**, daß die Entscheidungsmittel (16) weiterhin entscheiden, daß eine herzschrittmacherverursachte Tachykardie nicht vorliegt, wenn der Wert der Korrelation unter einen zweiten vorgegebenen Wert fällt, der niedriger ist als der erste vorgegebene Wert.

4. Ein Herzschrittmacher nach einem der vorhergehenden Ansprüche, **dadurch gekennzeichnet**, daß die variablen Verzögerungsmittel dazu vorgesehen sind, die Verzögerung in Übereinstimmung mit einem vorgegebenen/programmierbaren Muster zu variieren.

5. Ein Herzschrittmacher nach Anspruch 4, **dadurch** **gekennzeichnet**, daß die Korrelationsberechnung für wenigstens zwei Herzzyklen durchgeführt wird.

6. Ein Herzschrittmacher nach irgend einem der vorhergehenden Ansprüche, **dadurch gekennzeichnet**, daß das gemessene Zeitintervall das Intervall zwischen aufeinanderfolgenden P-Wellen ist.

7. Ein Herzschrittmacher nach den Ansprüchen 5 - 6, **dadurch gekennzeichnet**, daß die Korrelationsberechnung ausgedehnt wird, um Zeitverzögerungen und Zeitmessungen für weitere Herzzyklen einzuschließen, wenn der Wert der Korrelation zwischen den ersten und zweiten vorgegebenen Wert fällt.

8. Ein Herzschrittmacher nach irgend einem der vorhergehenden Ansprüche, **dadurch gekennzeichnet**, daß die Berechnungsmittel (16):
den Mittelwert für die Zeitverzögerungen und gemessenen Zeitintervalle und die Abweichung von dem Mittelwert für jede Zeitverzögerung beziehungsweise jedes gemessene Zeitintervall berechnen;
die Abweichung für jede Zeitverzögerung und jedes korrespondierende gemessene Zeitintervall multiplizieren und die Produkte für alle Zeitverzögerungen und gemessenen Zeitintervalle zu einer reslutierenden Summe addieren;
die Summe der absoluten Werte der Abweichungen der Zeitverzögerungen berechnen;
die resultierende Summe durch die Summe der absoluten Werte dividieren.

9. Ein Herzschrittmacher nach Anspruch 8, **dadurch gekennzeichnet**, daß das gemessene Zeitintervall das Intervall zwischen aufeinanderfolgenden ventrikulären Stimulationsimpulsen und P-Wellen ist und daß die Entscheidungsmittel (16) immer dann entscheiden, daß eine herzschrittmacherverursachte Tachykardie vorliegt, wenn der Korrelationswert unter den ersten vorgegebenen Wert fällt.

10. Ein Herzschrittmacher nach Anspruch 9, **dadurch gekennzeichnet**, daß die Entscheidungsmittel (16) weiterhin entscheiden, daß keine herzschrittmacherverursachte Tachykardie vorliegt, wenn der Wert der Korrelation einen zweiten Wert übersteigt, der höher ist als der erste vorgegebene Wert.

## Revendications

1. Stimulateur (10) cardiaque comportant au moins des moyens (12) de détection destinés à détecter des événements auriculaires ; des moyens (14) de stimulation destinés à stimuler le ventricule ; des moyens (153) à retard variable destinés à produire un retard temporel variable entre la détection d'événements auriculaires et la stimulation du ventricule ; des moyens (16) de mesure du temps destinés à mesurer l'intervalle de temps qui s'écoule entre des signaux consécutifs parmi lesquels au moins l'un est produit par le coeur ; des moyens (16) de décision destinés à au moins décider qu'une tachycardie induite par un stimulateur cardiaque est présente, caractérisé par des moyens (16) de calcul destinés à calculer la corrélation entre le retard temporel et l'intervalle de temps mesuré ; et en ce que les moyens (16) de décision décident qu'une tachycardie induite par un stimulateur cardiaque est présente chaque fois que la valeur de la corrélation présente un écart par rapport à une première valeur prédéterminée.

2. Stimulateur cardiaque suivant la revendication 1, caractérisé en ce que les moyens (16) de décision décident qu'une tachycardie induite par un stimulateur cardiaque est présente chaque fois que la valeur de la corrélation dépasse la première valeur prédéterminée.

3. Stimulateur cardiaque suivant les revendications 1 et 2, caractérisé en ce que les moyens (16) de décision décident en outre qu'une tachycardie induite par un stimulateur cardiaque n'est pas présente lorsque la valeur de la corrélation tombe en dessous d'une seconde valeur prédéterminée qui est inférieure à la première valeur prédéterminée.

4. Stimulateur cardiaque suivant l'une quelconque des revendications précédentes, caractérisé en ce que les moyens (153) à retard variable sont disposés de manière à faire varier le retard suivant une configuration programmable/prédéterminée.

5. Stimulateur cardiaque suivant la revendication 4, caractérisé en ce que le calcul de corrélation est réalisé pendant au moins deux cycles cardiaques.

6. Stimulateur cardiaque suivant l'une quelconque des revendications précédentes, caractérisé en ce que l'intervalle de temps mesuré est l'intervalle de temps qui s'écoule entre deux ondes P consécutives.

7. Stimulateur cardiaque suivant les revendications 5 à 6, caractérisé en ce que le calcul de corrélation est étendu de manière à inclure des retards de temps et des mesures de temps pour des cycles cardiaques supplémentaires lorsque la valeur de la corrélation tombe entre les première et seconde valeurs prédéterminées.

8. Stimulateur cardiaque suivant l'une quelconque des revendications précédentes, caractérisé en ce que les moyens (16) de calcul :
- calculent la valeur moyenne pour les retards de temps et les intervalles de temps mesurés et l'écart par rapport à la valeur moyenne pour chaque retard de temps et chaque intervalle de temps mesuré, respectivement ;
- multiplient les écarts pour chaque retard de temps et chaque intervalle de temps mesuré correspondant et ajoutent les produits pour tous les retards de temps et intervalles de temps mesurés en une somme résultante ;
- calculent la somme des valeurs absolues des écarts des retards de temps ;
- divisent la somme résultante par la somme des valeurs absolues.

9. Stimulateur cardiaque suivant la revendication 8, caractérisé en ce que l'intervalle de temps mesuré est l'intervalle de temps qui s'écoule entre des impulsions de stimulation ventriculaires et des ondes P consécutives, et en ce que les moyens (16) de décision décident qu'une tachycardie induite par un stimulateur cardiaque est présente chaque fois que la valeur de corrélation tombe en dessous de la première valeur prédéterminée.

10. Stimulateur cardiaque suivant la revendication 9, caractérisé en ce que les moyens (16) de décision décident en outre qu'une tachycardie induite par un stimulateur cardiaque n'est pas présente lorsque la valeur de la corrélation dépasse une seconde valeur qui est supérieure à la première valeur prédéterminée.
